# EUROPEAN PATENT SPECIFICATION

(11) **EP 1 624 880 B1**
(45) Date of publication and mention of the grant of the patent: **19.12.2018**
(21) Application number: 04750527.6
(22) Date of filing: 08.04.2004
(51) Int. Cl.: A61K 36/18

(54) **ANNATTO EXTRACT COMPOSITIONS, INCLUDING GERANYL GERANIOLS FOR USE IN THE DECREASE IN THE BLOOD LEVEL OF TRIGLYCERIDE**
ANNATTO-EXTRAKT-ZUSAMMENSETZUNGEN MIT GERANYL-GERANIOLEN ZUR VERWENDUNG BEI DER SENKUNG DER TRIGLYCERID-KONZENTRATION IM BLUT
COMPOSITIONS D'EXTRAIT DE ROCOU CONTENANT DES GERANYLGERANIOLS POUR UTILISATION DANS LA DIMINUTION DE NIVEAU SANGUIN DE TRIGLYCERIDE

(30) Priority: 08.04.2003 US 461612 P; 10.12.2003 US 528353 P
(43) Date of publication of application: 15.02.2006
(60) Divisional application: 11195385.7
(73) Proprietor: Tan, Barrie, Amherst, MA 01002 (US)
(72) Inventor: Tan, Barrie, Amherst, MA 01002 (US)
(74) Representative: Vossius & Partner Patentanwälte Rechtsanwälte mbB
(86) International application number: PCT/US2004/012542
(87) International publication number: WO 2004/096137

(56) References cited:
- WO-A1-00/71531
- WO-A1-99/66929
- WO-A2-03/047528
- US-A- 5 084 292
- US-A- 5 756 109
- US-A- 6 083 979
- US-B1- 6 350 453
- ANONYMOUS: "Annatto (Bixa orellana)" RAINTREE NUTRITION (TROPICAL PLANT DATABASE), [Online] 2005, pages 1-7, XP002538018 Retrieved from the Internet: URL:http://www.rain-tree.com/annato.htm> [retrieved on 2009-07-21]
- TANAKA Y ET AL: "Separation of geranylgeraniol isomers by high-performance liquid chromatography and identification by <13>C nuclear magnetic resonance spectroscopy" JOURNAL OF CHROMATOGRAPHY, ELSEVIER SCIENCE PUBLISHERS B.V. AMSTERDAM, NL, vol. 347, 1 January 1985 (1985-01-01), pages 275-283, XP025492895 ISSN: 0021-9673 [retrieved on 1985-01-01]
- MU Y ET AL: "Coupling of Isoprenoid Triflates with Organoboron Nucleophiles: Synthesis of all-trans-Geranylgeraniol" TETRAHEDRON LETTERS, ELSEVIER, AMSTERDAM, vol. 36, no. 32, 7 August 1995 (1995-08-07), pages 5669-5672, XP004027574 ISSN: 0040-4039
- TAKAHASHI N ET AL: "Dual action of isoprenols from herbal medicines on both PPARgamma and PPARalpha in 3T3-L1 adipocytes and HepG2 hepatocytes" FEBS LETTERS, ELSEVIER, AMSTERDAM, NL, vol. 514, no. 2-3, 13 March 2002 (2002-03-13), pages 315-322, XP004347719 ISSN: 0014-5793
- Asaf A Qureshi ET AL: "Dose-dependent suppression of serum cholesterol by tocotrienol-rich fraction (TRF 25 ) of rice bran in hypercholesterolemic humans", Atherosclerosis, vol. 161, 1 January 2002 (2002-01-01), pages 199-207, XP055263812,
- JONDIKO I J O ET AL: "Terpenoids and an apocarotenoid from seeds of Bixa orellana", PHYTOCHEMISTRY, PERGAMON PRESS, GB, vol. 28, no. 11, 1 January 1989 (1989-01-01), pages 3159-3162, XP026620780, ISSN: 0031-9422, DOI: 10.1016/0031-9422(89)80298-5 [retrieved on 1989-01-01]
- SACHAN K ET AL: "Bixa orellana L.- A dye yielding plant having medicinal properties", MEDICINAL & AROMATIC PLANTS ABSTRACTS, SCIENTIFIC PUBLISHERS, SCIENTIFIC PUBLISHERS, NEW DELHI - INDIA, vol. 25, no. 2, 1 April 2003 (2003-04-01), XP018013015, ISSN: 0250-4367
- D. Mahabir ET AL: "Use of medicinal plants for diabetes in Trinidad and Tobago", Revista Panamericana de Salud Publica, vol. 1, no. 3, 1 January 1997 (1997-01-01) , pages 174-179, XP055326083, US ISSN: 1020-4989, DOI: 10.1590/S1020-49891997000300002
- Anonymous: "Annatto - Bixa orellana", RAINTREE NUTRITION (TROPICAL PLANT DATABASE), 1 December 2002 (2002-12-01), page 5 pp., XP055326273, Retrieved from the Internet: URL:https://web.archive.org/web/2002120110 1723/http://www.rain-tree.com/annato.htm [retrieved on 2016-12-06]
- DANIELA DE ARAÚJO VILAR ET AL: "Traditional Uses, Chemical Constituents, and Biological Activities of Bixa orellana L.: A Review", THE SCIENTIFIC WORLD JOURNAL, vol. 10, no. 3, 1 January 2014 (2014-01-01), pages 145-11, XP055324929, ISSN: 2356-6140, DOI: 10.1007/978-3-642-66896-8_16
- E.Y. MORRISON AND M.E. WEST: "The effect of Bixa orallana (Annatto) on blood sugar levels in the naesthetized dog", WEST INDIAN MEDICAL JOURNAL, vol. 34, 1985, pages 38-42, XP55324929,

## Description

### Background of the Invention

### References

Adachi, J. and G. Ioannidis (2000). "Glucocorticoid-induced osteoporosis." Drug Development Research 49: 120-134.
Amin, D., R. Rutledge, et al. (1997). "RPR 107393, a potent squalene synthase inhibitor and orally effective cholesterol-lowering agent: comparison with inhibitors of HMG-CoA reductase." J Pharmacol Exp Ther. 281(2): 746-752.
Ayanian, J., C. Fuchs, et al. (1988). "Lovastatin and rhabdomyolysis." Ann Intern Med. 109: 682-683.
Baker, S. and M. Tarnopolsky (2001). "Statin myopathies: pathophysiologic and clinical perspectives." Clin Invest Med. 24(5): 258-272.
Ballantyne, C., A. Corsini, et al. (2003). "Risk for myopathy with statin therapy in high-risk patients." Arch. Intern. Med. 163(5): 553-564.

Steroids are widely used and the most common among them is prednisone. Corticosteroids are used for many inflammatory diseases including but not limited to arthritis, connective tissue disease, asthma, and in heart transplant patients. These corticosteroids have several side effects including rapid loss of bone mass in the first year of use, as high as 15% of patients develop vertebral fractures (Adachi and Ioannidis 2000), loss of bone mineral density even at very low doses, e.g. prednisone at 5 mg/day (Saito, Davis et al. 1995), and a high rate of steroid-induced osteoporosis, higher than osteoporosis in post menopausal women (Miller 2001). To prevent and reverse corticosteroid-induced osteoporosis, bisphosphonates has become the best drug candidate.

The role of Vitamin E in exercise is well known. Muscle damage can occur during exhaustive exercise, even in highly trained athletes. Furthermore, since the body's Vitamin E consumption increases with the amount of exercise, high amounts of Vitamin E are needed for endurance training and for membrane lipid oxidation protection during strenuous exercise (VERIS 1989).

Statins and bisphosphonates can increase the risk of adverse ocular side effects including cataracts (Schlienger, Haefeli et al. 2001). Statins increase the mRNA and the protein mass of HMGR, which translates to an over expression of cholesterol biosynthesis in intact lens (Cenedella 1995; Cenedella 1997). It is suggested that IP products might prevent lens opaqueness, cataract, and lens cholesterol deposition. Cataract removal remains the most common surgery in the US (more than half million per year). The occurrence of cataracts approachs 50% for those 75 years or older. The protective use of Vitamin E against cataract development is well recognized (VERIS 1990). Vitamin E tocotrienols and tocopherols are both powerful antioxidants. However, only tocotrienols, especially delta- and gamma-tocotrienols have been shown to down regulate the mRNA and reduce the protein mass of HMGR.

### Summary of the Invention

The invention relates to a composition comprising tocotrienols and geranyl geraniols, obtainable from a by-product oily phase of annatto colorant from annatto seeds, wherein the concentration of annatto colorant in the by-product is less than 2% by weight where the tocotrienols are essentially in delta and gamma isomer forms and where the tocotrienols have a delta-to-gamma isomer ratio between 1:100 and 100:1 and where the geranyl geraniols include cis and trans isomer forms and where the geranyl geraniols have a trans-to-cis isomer ratio between 1:100 and 100:1, for use in the decrease in the blood level of the triglyceride which has an effect selected from the group consisting of reversal of insulin resistance, metabolic syndrome, prediabetes, diabetes and diabetes-related cardiovascular disease. In a preferred embodiment, the composition is for use in a method to reverse insulin resistance.

The trans-to-cis isomer ratio of geranyl geraniols is between 1:100 to 100:1. Preferably, the trans-to-cis isomer ratio of geranyl geraniols is between 1:5 to 5:1. More preferably, the trans-to-cis isomer ratio of geranyl geraniols is > 5:1.

The delta-to-gamma ratio of tocotrienols is between 1:100 to 100:1. Preferably, the delta-to-gamma ratio of tocotrienols is between 1:5 to 5:1. More preferably, the delta-to-gamma ratio of tocotrienols is > 5:1.
Disclosed herein are:
A method to benefit the health of an animal,
comprising administering annatto extract containing geranyl geraniols and increasing an amount of a biological factor to provide or restore a function selected from the group consisting of mitochondrial respiration, lipid protection, heme, DL-glycosylated and GG-prenylated proteins. Preferred is a method where the biological factor is selected from the group consisting of CoQ10, dolichol (DL), and porphyrin syntheses.

A method of reversing isoprenoid pool deprivation, comprising administering annatto extract containing geranyl geraniols.

A method to increase CoQ10, comprising administering annatto extract containing geranyl geraniols and anabolically increasing the endogenous *de novo* synthesis of CoQ10.

A method to reverse insulin resistance, comprising administering annatto extract containing geranyl geraniols and potentiating insulin.

A method to reverse insulin resistance, comprising administering annatto extract containing geranyl geraniols and potentiating insulin, further comprising lowering the risk of a disease selected from the group consisting of CVD, T2DM, hypertension, PCOS and fatty liver disease.

A method to activate the nuclear transcription factor PPAR, comprising administering annatto extract containing geranyl geraniols and causing an effect selected from the group consisting of increasing cellular uptake, increasing mitochondrial uptake, increasing beta-oxidation catabolism, increasing triglyceride metabolism, decreasing plasma FFA, decreasing triglycerides, reducing hyperglycemia, reducing hyperinsulinemia, enhancing insulin sensitivity and lowering insulin resistance.

A method to inhibit *de novo* biosynthesis of fatty acids, comprising administering annatto extract containing geranyl geraniols and deactivating of SREBP-1 expression. Preferred is a method to inhibit *de novo* biosynthesis of fatty acids, comprising administering annatto extract containing geranyl geraniols and deactivating of SREBP-1 expression, where the deactivating of SREBP-1 expression causes a decrease in TG.

A method to inhibit *de novo* biosynthesis of fatty acids, comprising administering annatto extract containing geranyl geraniols and deactivating of SREBP-1 expression, where the deactivating of SREBP-1 expression is in organs selected from the group consisting of liver, adipose and skeletal muscle.

A method to inhibit *de novo* biosynthesis of fatty acids, comprising administering annatto extract containing geranyl geraniols and deactivating of SREBP-1 expression, where there is a decrease in the plasma levels of factors selected from the group consisting FFA, TG, LDL, total cholesterol.

A method to inhibit *de novo* biosynthesis of fatty acids, comprising administering annatto extract containing geranyl geraniols and deactivating of SREBP-1 expression, where there is a decrease in fat storage.

A method to reduce drug toxicities, comprising administering annatto extract containing geranyl geraniols and reducing the myotoxicities of drugs selected from the group consisting of statins, cyclosporines, fibrates, bisphosphonates, and farnesol transferase inhibitors.

A method to reverse insulin resistance, metabolic syndrome or diabetes, comprising administering annatto extract containing geranyl geraniols, and increasing Glut 4 and decreasing TG.
Disclosed herein are also:
A composition of annatto extract, which
includes geranyl geraniols and tocotrienols, that increases the *de novo* biosyntheses of all subsequent intermediate isoprenoid pool and distal products.

A composition of annatto extract with geranyl geraniols that has both trans geranyl geraniol and 2-4 cis geranyl geraniols where the trans-to-cis ratio is 1:100 to 100:1. Preferred is a composition of annatto extract with geranyl geraniols that has both trans geranyl geraniols and 2-4 cis geranyl geraniols where the trans-to-cis ratio is 1:5 to 5:1. More preferred is a composition of annatto extract with geranyl geraniols that has both trans geranyl geraniols and 2-4 cis geranyl geraniols where the trans-to-cis ratio is > 5:1.

A composition of annatto extract with T3 that has both delta- T3 and gamma- T3, where the delta-to-gamma ratio is 1:100 to 100:1. In preferred embodiment, the invention is drawn to a composition of annatto extract with T3 that has both delta-T3 and gamma-T3, where the delta-to-gamma ratio is 1:5 to 5:1. In a more preferred embodiment, the invention is drawn to a composition of annatto extract with T3 that has both delta- T3 and gamma- T3, where the delta-to-gamma ratio is >5:1.

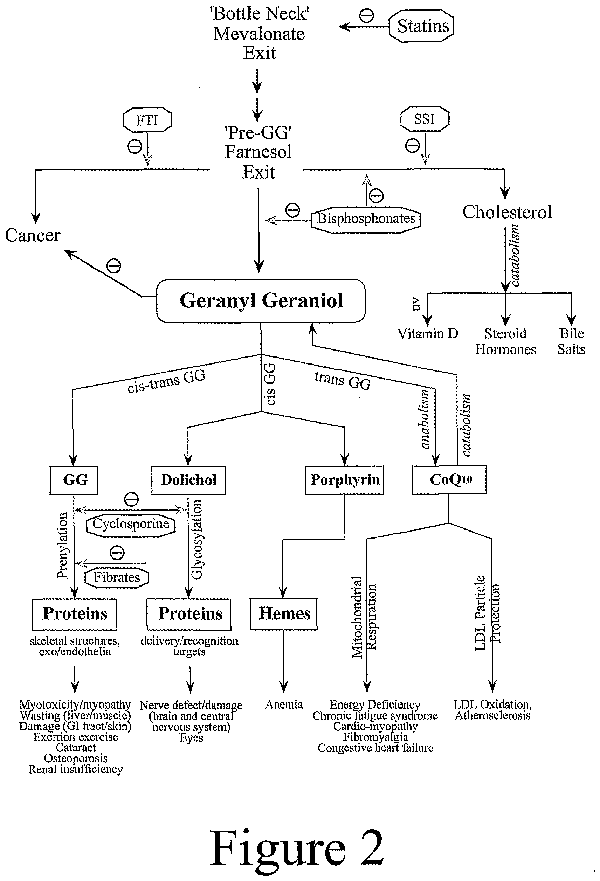

A composition containing geranyl geraniol, especially an unique cis-GG and trans-GG ratio that raises CoQ10, dolichol (DL), and porphyrin syntheses, and thereby provides and/or restores mitochondrial respiration and lipid protection, heme, DL-glycosylated and GG-prenylated proteins, respectively, and described in Figure 2.

A composition of annatto extract containing GG which reverses IP deprivation from drug-induced and non drug-induced maladies. In an alternative embodiment, the invention is drawn to a method of administering an annatto extract containing GG and reversing IP deprivation from drug-induced and non drug-induced maladies.

A composition of an annatto extract containing GG that anabolically increases the endogenous *de novo* synthesis of CoQ10 via GG elongation/prenylation of side chain and conversely CoQ10 catabolically increases the endogenous *de novo* synthesis of GG via CoQ10 beta-oxidation.

A composition of annatto extract with GG that potentiates insulin, which therefore promotes insulin sensitivity, and/or reverses insulin resistance in normal weight and overweight/obese subjects, and in both sexes. Preferred is a composition of annatto extract with GG that potentiates insulin and/or reverses insulin resistance that reduces the risk of CVD, T2DM, hypertension, PCOS and fatty liver disease. More preferred is a composition of annatto extract with GG that potentiates insulin and/or reverses insulin resistance that activates the nuclear transcription factor PPAR (γ, α, δ, or mixed) expression. More preferred is a composition of annatto extract with GG that potentiates insulin and/or reverses insulin resistance that activates the nuclear transcription factor PPAR (γ, α, δ, or mixed) expression, and carries out the metabolism-effected increase of cellular and/or mitochondrial uptake and beta-oxidation catabolism. More preferred is a composition of annatto extract with GG that potentiates insulin and/or reverses insulin resistance that activates the nuclear transcription factor PPAR (γ, α, δ, or mixed) expression, and carries out the metabolism-effected increase of cellular and/or mitochondrial uptake and beta-oxidation catabolism, and then increases triglyceride metabolism. Even more preferred is a composition of annatto extract with GG that potentiates insulin and/or reverses insulin resistance that activates the nuclear transcription factor PPAR (γ, α, δ, or mixed) expression, and carries out the metabolism-effected increase of cellular and/or mitochondrial uptake and beta-oxidation catabolism, and then increases triglyceride metabolism, which then decreases plasma FFA and triglyceride. Even more preferred is a composition of annatto extract with GG that potentiates insulin and/or reverses insulin resistance that activates the nuclear transcription factor PPAR (γ, α, δ, or mixed) expression, and carries out the metabolism-effected increase of cellular and/or mitochondrial uptake and beta-oxidation catabolism, and then increases triglyceride metabolism, which then decreases plasma FFA and triglyceride, and resulting in a reduction of hyperglycemia, HI, enhancement of IS and/or lowering of IR states. Most preferred is a composition of annatto extract with GG that potentiates insulin and/or reverses insulin resistance that activates the nuclear transcription factor PPAR (γ, α, δ, or mixed) expression, and carries out the metabolism-effected increase of cellular and/or mitochondrial uptake and beta-oxidation catabolism, and then increases triglyceride metabolism, which then decreases plasma FFA and triglyceride, and resulting in a reduction of hyperglycemia, HI, enhancement of IS and/or lowering of IR states, where the PPAR (γ, α, δ, or mixed) activation is expressed in numerous organs and tissues in the body.

A composition of annatto extract or annatto extract containing GG that deactivates SREBP-1 expression, and inhibits the *de novo* biosynthesis of fatty acid. Preferred is a composition of annatto extract or annatto extract containing GG that deactivates SREBP-1 expression, and inhibits the *de novo* biosynthesis of fatty acid, and results in a decrease of TG. Preferred is a composition of annatto extract or annatto extract containing GG that deactivates SREBP-1 expression in various organs, including liver, adipose and skeletal muscle. More preferred is a composition of annatto extract or annatto extract containing GG that deactivates SREBP-1 and activates PPAR to control the synthesis and/or metabolism of FFA/TG. More preferred is a composition of annatto extract or annatto extract containing GG that deactivates SREBP-1 and activates PPAR to control the synthesis and/or metabolism of FFA/TG, and causes a decrease of lipids in the plasma. Most preferred is a composition of annatto extract or annatto extract containing GG that deactivates SREBP-1 and activates PPAR to control the synthesis and/or metabolism of FFA/TG, and causes a decrease of lipids in the plasma, and the lipids include LDL and total cholesterol. Most preferred is a composition of annatto extract or annatto extract containing GG that deactivates SREBP-1 and activates PPAR to control the synthesis and/or metabolism of FFA/TG, and causes a decrease of lipids in the plasma, and the lipids include LDL and total cholesterol, and the animal reduces fat storage and/or loses weight.
Disclosed herein is also:
A method to reduce drug side effects comprising administering an annatto extract and reducing drug toxicities. Preferred is a method to reduce drug side effects comprising administering an annatto extract and reducing myotoxicities. More preferred is a method to reduce drug side effects comprising administering an annatto extract and reducing myotoxicities, where the myotoxicities are selected from the group consisting of myalgia, myopathy, rhabdomyolysis, and myonecrosis. More preferred is a method to reduce drug side effects comprising administering an annatto extract and reducing myotoxicities, where the myotoxicities are selected from the group consisting of myalgia, myopathy, rhabdomyolysis, and myonecrosis, and are caused by drugs selected from the group of statins, cyclosporines, fibrates, farnesyl transferase inhibitor, and bisphosphonates. More preferred is a method to reduce drug side effects comprising administering an annatto extract and reducing myotoxicities, where the drug induced toxicities are related to the inhibition of GG, DL, heme, and CoQ10.
Disclosed is a method to reverse insulin resistance, metabolic syndrome and/or diabetes comprising administering an annatto extract or annatto extract containing GG, that reverses and/or salvages Glut 4 inhibition. Preferred is
a method to reverse insulin resistance, metabolic syndrome and/or diabetes comprising administering an annatto extract or annatto extract containing GG, that reverses and/or salvages Glut 4 inhibition, where the levels of Glut 4 increases and/or TG decreases.
Disclosed herein are also:
A method of correcting nutritional maladies
and/or cellular dysmetabolism, comprising administering an annatto extract or annatto extract containing GG, and inhibiting HMGR and/or lowering cholesterol synthesis.
Preferred is a method of correcting nutritional maladies and/or cellular dysmetabolism, comprising administering an annatto extract or annatto extract containing GG, and inhibiting HMGR and/or lowering cholesterol synthesis, and where the inhibiting of HMGR and/or lowering of cholesterol synthesis, does not inhibit endogenous CoQ10 synthesis. More preferred is
a method of correcting nutritional maladies and/or cellular dysmetabolism, comprising administering an annatto extract or annatto extract containing GG, and inhibiting HMGR and/or lowering cholesterol synthesis, and where the inhibiting of HMGR and/or lowering of cholesterol synthesis, does not inhibit endogenous CoQ10 synthesis and does salvage plasma CoQ10. More preferred is
a method of correcting nutritional maladies and/or cellular dysmetabolism, comprising administering an annatto extract or annatto extract containing GG, and inhibiting HMGR and/or lowering cholesterol synthesis, and protecting LDL from oxidation and/or increasing cellular ATP energy production. Most preferred is
a method of correcting nutritional maladies and/or cellular dysmetabolism, comprising administering an annatto extract or annatto extract containing GG, and inhibiting HMGR and/or lowering cholesterol synthesis, and decreasing TG, prediabetes and/or diabetes.

A method reducing the effect of maladies comprising the administering of an annatto extract or annatto extract containing GG, wherein GG's distal and intermediate products, and proteins reverse maladies and dysfunctions selected from the group consisting of the central nervous system, GI track, skin (endothelial and exothelial), eye, muscle, blood/heme, and kidney.

A method of inhibiting cancer growth, comprising the administering of an annatto extract or annatto extract containing GG.

A method of supplementation, comprising the administering of an annatto extract or annatto extract containing GG, and preventing statin toxicities, increasing CoQ10, protecting LDL, lowering cholesterol and/or improving endothelial functions.

A method of therapy, comprising the administering of an annatto extract or annatto extract containing GG as a drug adjunct for cancer therapy. Preferred is a method of therapy, comprising the administering of an annatto extract or annatto extract containing GG as a drug adjunct for FTI therapy.

A method of supplementation, comprising the administering of an annatto extract or annatto extract containing GG and reversing myotoxicities.

A method of supplementation, comprising the administering of an annatto extract or annatto extract containing GG in conjunction with CoQ10, and providing *ex vivo* and *in vivo* GG substrate or GG alone for treating prostate cancer and/or breast cancer.

A method of supplementation, comprising the administering of an annatto extract or annatto extract containing GG and inhibiting cancer growth where GG involvement is not required in protein prenylation.

A method of supplementation, comprising the administering of an annatto extract or annatto extract containing GG to patients with renal insufficiency and/or kidney dialysis.

A method of supplementation, comprising the administering of an annatto extract or annatto extract containing GG to transplant recipients and reversing and/or minimizing myopathy and rhabdomyolysis, where the supplement is used as an adjunct therapy to calcineurin inhibitors and statins. Preferred is
a method of supplementation, comprising the administering of an annatto extract or annatto extract containing GG to transplant recipients and reversing and/or minimizing myopathy and rhabdomyolysis, where the supplement is used as an adjunct therapy to cyclosporine.

A method of supplementation, comprising the administering of an annatto extract or annatto extract containing GG where drugs deplete GG and reduce protein prenylation, causing myotoxicity.

A method of supplementation, comprising the administering of an annatto extract or annatto extract containing GG, and abrogating the effects of insufficient CYP3A4 processing of statin (in mono- or combo- therapies) and/or reversing the compromise on the vascular system.

A method of supplementation, comprising the administering of an annatto extract or annatto extract containing GG, and using the annatto extract or annatto extract containing GG as an adjunct to mono- and combo-therapies including fibrates. Preferred is a method of supplementation, comprising the administering of an annatto extract or annatto extract containing GG, and using the annatto extract or annatto extract containing GG as an adjunct to mono- and combo-therapies including fibrates, and with prediabetes, diabetes, and/or hypertriglyceridemia patients.

A method of supplementation, comprising the administering of an annatto extract or annatto extract containing GG, and treating lipidemia of normal or overweight/obese patients. Preferred is
a method of supplementation, comprising the administering of an annatto extract or annatto extract containing GG, and treating lipidemia of normal or overweight/obese patients, and decreasing the level of TG.

A method of supplementation, comprising the administering of an annatto extract or annatto extract containing GG, and activating the nuclear transcription factor PPAR (γ, α, δ or mixed) and carrying out metabolic effects similar to TZDs and fibrates, in various tissues of common sites (adipose, skeletal muscle, and kidney, macrophage, VSMC, endothelial cell) and in various tissues of different sites for PPARγ (heart, gut) and PPARα (liver).

A composition of annatto extract or annatto extract containing GG for a coating to prevent "pill esophagitis", where GG is in a film-coat on compressed tablets, softgel gelatin, hard gel two-piece gelatin, beads, granules, and/or liquid coats.

A method of supplementation, comprising the administering of an annatto extract or annatto extract containing GG, and promoting general upper GI health.

A method of supplementation, comprising the administering of an annatto extract or annatto extract containing GG, and using annatto extract or annatto extract containing GG as an adjunct with combined steroid and bisphosphonate medications.

A composition of annatto extract or annatto extract containing GG, for supplementation during exercise. Preferred is a composition of annatto extract or annatto extract containing GG for supplementation during heavy training and/or exertion exercise. More preferred is a composition of annatto extract or annatto extract containing GG that further contains tocotrienols for supplementation during heavy training and/or exertion exercise.

A method of supplementation, comprising the administering of an annatto extract or annatto extract containing GG, and reducing drug-induced cataract formation. Preferred is a method of supplementation, comprising the administering of an annatto extract or annatto extract containing GG, and the annatto extract further containing tocotrienols, and reducing drug-induced cataract formation.

A composition of annatto extract or annatto extract containing GG, and the composition further comprising formulation with other synergistic and/or useful non-drug vitamin and mineral nutrients. Preferred is a composition of annatto extract or annatto extract containing GG, and the composition further comprising formulation with niacin, other B Vitamins, and iron.
Preferred is a composition of annatto extract or annatto extract containing GG, and the composition further comprising formulation with ubiquinone and/or idebenone.
Preferred is a composition of annatto extract or annatto extract containing GG, and the composition further comprising formulation with plaunotol and/or micro-protective GI track support nutrients to provide support for the entire "upper-lower" alimentary canal.
Preferred is a composition of annatto extract or annatto extract containing GG, and the composition further comprising formulation with tocotrienols and tocotrienol rich fractions to promote beneficial effects in the nervous and/or immune system. More preferred is
a composition of annatto extract or annatto extract containing GG, and the composition further comprising formulation with tocotrienols and tocotrienol rich fractions, where the tocotrienol rich fractions are from palm and rice. More preferred is a composition of annatto extract or annatto extract containing GG, and the composition further comprising formulation with tocotrienols and tocotrienol rich fractions to promote beneficial effects in the autonomal nervous system.
Preferred is a composition of annatto extract or annatto extract containing GG, and the composition further comprising formulation with non-vitamin endogenous nutrients, which include but not limited to, carnitines, CoQ10, alpha lipoic acid, omega 3 fatty acids, linseed/flaxseed oil, creatine, SOD, and NADH.
Preferred is a composition of annatto extract or annatto extract containing GG, and the composition further comprising formulation with drugs to lessen or eliminate the drug toxicities. More preferred is a composition of annatto extract or annatto extract containing GG, and the composition further comprising formulation with drugs to lessen or eliminate the drug toxicities, where the drugs are selected from the group consisting of statins, bisphosphonate, fibrates, cyclosporines, niacin, warfarin/coumadin, antifungals, and antibiotics.

### Brief Description of the Drawings

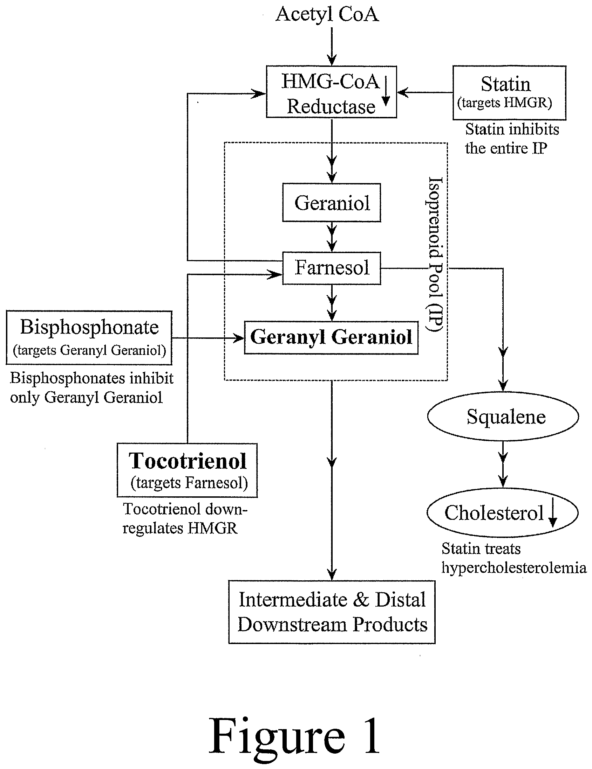

Figure 1 illustrates the Mevalonate Acid General Pathway.
Figure 2 illustrate the GG downstream distal products and upstream relationships with hatched boxes representing the distal products for GG and octagonal boxes representing the drugs that inhibit specific pathways.
Figure 3 illustrates the effect of annatto extract compositions on LDL and CoQ10.
Figure 4 illustrates the endogenous metabolism of CoQ10 and GG via respective exogenous GG and CoQ10.

### Detailed Description Of The Invention and Preferred Embodiment

### Detailed Description of the Preferred Embodiment

The subject-matter of the invention is a composition comprising tocotrienols and geranyl geraniols, obtainable from a by-product oily phase of annatto colorant from annatto seeds, wherein the concentration of annatto colorant in the by-product is less than 2% by weight where the tocotrienols are essentially in delta and gamma isomer forms and where the tocotrienols have a delta-to-gamma isomer ratio between 1:100 and 100:1 and where the geranyl geraniols include cis and trans isomer forms and where the geranyl geraniols have a trans-to-cis isomer ratio between 1:100 and 100:1, for use in the decrease in the blood level of the triglyceride which has an effect selected from the group consisting of reversal of insulin resistance, metabolic syndrome, prediabetes, diabetes and diabetes-related cardiovascular disease.

In a preferred embodiment, the composition contains annatto extract with geranyl geraniols, where the geranyl geraniols have a trans-to-cis isomer ratio between 1:5 to 5:1. In a more preferred embodiment, the composition contains annatto extract with geranyl geraniols, where the geranyl geraniols have a trans-to-cis isomer ratio 1:1.

In a preferred embodiment, the composition contains annatto extract with tocopherol-free C-5 unsubstituted tocotrienols, where the tocotrienols extract have a delta-to-gamma ratio between 1:5 to 5:1. In a preferred embodiment, the composition contains annatto extract with tocopherol-free C-5 unsubstituted tocotrienols, where the tocotrienols extract have a delta-to-gamma ratio 1:1.
In one embodiment, a composition contains annatto extract with geranyl geraniols and tocopherol-free C-5 unsubstituted tocotrienols. In a more preferred embodiment, the composition contains annatto extract with geranyl geraniols, tocopherol-free C-5 unsubstituted tocotrienols, and inactive and/or active ingredients.

### Examples

The following examples describe embodiments of the invention. Other embodiments within the scope of the claims herein will be apparent to one skilled in the art from consideration of the specification or practice of the invention as disclosed herein. It is intended that the specification, together with the examples, be considered to be exemplary only.

### Example 1 (for reference)

### DRUG AND NON-DRUG INDUCED MYOPATHIES VIA GG INHIBITION

In general, any process that depletes the IP, and in particular the administration of GG is the subject of this AEC invention.

The administration of AEC and GG reverses the effect of GG depletion by drug and non-drug induced myopathies. Also, the administration of GG and AEC containing GG during exercise, particularly in heavy training and exertion exercise reverses the effects of myopathies in animals. An unique application of AEC containing GG is to mix it with tocotrienols, including AEC containing tocotrienols, for heavy training and exertion exercise. There are simultaneous benefits of muscle/protein repair by GG and muscle/oxidation protection by tocotrienols.

### Example 2 (for reference)

### CoQ10

An average of 20% increase (1.01 to 1.20 µg/mL) in plasma CoQ10 was observed in patients taking AEC containing 20 mg of GG per day. This is equivalent to an exogensous CoQ10 supplementation of 20 mg per day to those knowledgeable in the art. In another investigation involving a single patient, the subject's endogenous CoQ10 rose by 70% from a plasma baseline level of 0.86 µg/mL to 1.47 µg/mL after 3 months of supplementation of AEC containing 27 mg of GG per day. This was the equivalent to an exogenous CoQ10 supplementation of about 35-70 mg/day to those knowledgeable in the art. After the AEC supplementation, the patient experienced increased energy and no longer suffered from chronic fatigue. This outcome was due to the utility of GG in the *de novo* biosynthesis of endogenous CoQ10.

**Table 1. Effect of annatto extratct supplementation on plasma CoQ10**

| | Plasma CoQ10 (µg/ml) | | |
|---|---|---|---|
| Subject | Control | After | Change (%) |
| 1 | 1.03 | 1.21 | 17.5 |
| 2 | 1.09 | 1.34 | 22.9 |
| 3 | 0.97 | 1.06 | 9.3 |
| 4 | 1.12 | 1.35 | 20.5 |
| 5 | 0.86 | 1.06 | 23.3 |
| Mean | 1.02 | 1.20 | 18.8 |

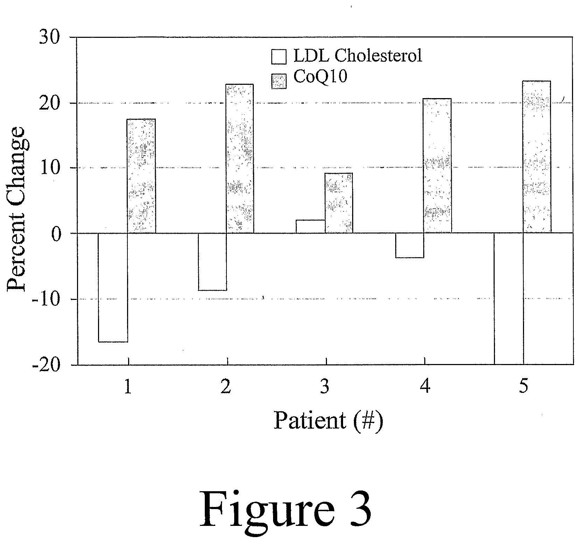

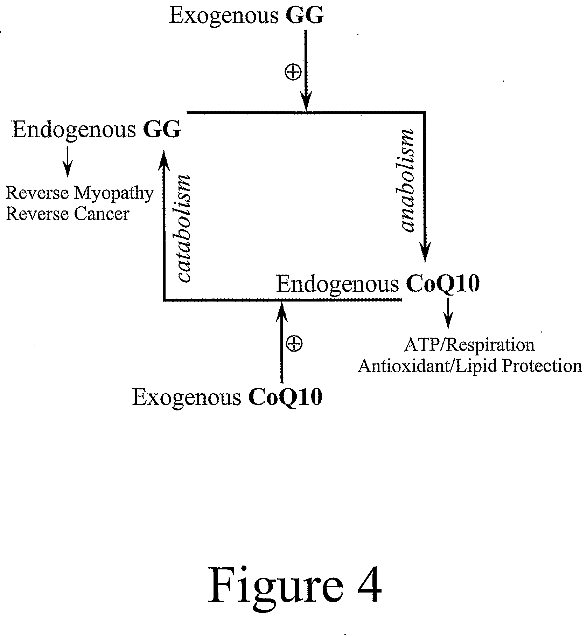

Table 1 and Figure 3 show the results of a study where the subjects took 20 mg/day of GG. According to the analytical method described in earlier section for anabolic conversion of GG to CoQ10, this is equivalent to the a theoretical conversion maximum of 30 mg/day (20 x 150/100) of CoQ10. Likewise, the 27 mg of GG/day consumed by the previous subject is equivalent to taking 40 mg CoQ10/day. Supplementation of GG for anabolic CoQ10 synthesis and supplementation CoQ10 for catabolic GG synthesis are reversible pathways and they are illustrated in Figure 4.

Exogenously supplied GG raises the plasma CoQ10 to similar levels achieved by exogenous CoQ10, and the increase in plasma levels of 20 to 70% are illustrative. Higher doses of GG will cause the plasma CoQ10 to rise further to levels typically reached by humans (i.e., 2 to 5 folds above baseline levels) who take 30-1000 mg CoQ10/day, or more typically 100-300 mg CoQ10/day. High exogenous doses of CoQ10 is often required to achieve therapeutic plasma levels since supplemental CoQ10 has attendant problems with bioavailability in that less than 5% is absorbed. Supplementation of CoQ10 raises the gut-to-blood CoQ10 level while supplementation of AEC causes cell-to-blood rise of CoQ10. This represents the first reported endogenously available CoQ10 from GG and from AEC containing GG.

### Example 3 (for reference)

### HYPERCHOLESTEROLEMIA

In borderline overweight volunteers (Table 2) on AEC, the T3-affected LDL drop corresponded with an increase in CoQ10 (Table 1 and Figure 3). AEC treats hypercholesterolemia without decreasing CoQ10. In fact, the CoQ10 level rises (20%) when supplemented with AEC, affording additional antioxidant protection to LDL particles by CoQ10.

**Table 2. Vital Statistics of Subjects**

| Subject | Height (ft in.) | Weight (lb) | Age | BMI (kg/m²) |
|---|---|---|---|---|
| 1 | 5' 6" | 156 | 26 | 25.2 |
| 2 | 6' 1" | 190 | 22 | 25.1 |
| 3 | 5" 7" | 143 | 50 | 22.4 |
| 4 | 6' 2" | 204 | 30 | 26.3 |
| 5 | 5" 6" | 156 | 26 | 26.7 |

Administration of AEC prevents statin toxicities, increases CoQ10, protects the LDL, lowers cholesterol and improves endothelial functions.

### Example 4 (for reference)

### CANCER

GG is given as a drug adjunct for cancer therapy in general and for FTI therapy in particular.

Supplementation with GG in AEC reverses myotoxicities. GG is not toxic to untransformed cells or to normal cells and is used as a statin adjunct in cancer therapy. CoQ10 catabolizes to GG and other smaller molecular weight substrates, which in turn inhibit the F- prenylated proteins.

GG is used alone or in conjunction with CoQ10 to provide *ex vivo* and *in vivo* GG substrate for prostate and breast cancer treatment.

AEC containing GG inhibits cancer growth where regardless of involvement of protein prenylation.

### Example 5 (for reference)

### RENAL INSUFFICIENCY:

GG and AEC are used in general and as an adjunct to the drugs used by kidney dialysis and renal insufficient patients.

### EXAMPLE 6 (for reference)

### ORGAN TRANSPLANT

GG and AEC are used to reverse and/or minimize the serious myopathy and rhabdomyolysis of graft recipients, where GG and AEC are used as adjunct therapy to calcineurin inhibitors (cyclosporine in particular) and statins.

### Example 7 (for reference)

### MYOTOXICITIES

Administration of GG reverses the side effect of drugs with a "common mechanism" of GG-depletion that causes a reduction of protein prenylation, which leads to myotoxicity. Supplementation with AEC abrogates the effects of insufficient CYP3A4 processing of statin (in mono- or combo- therapies) and reverses the compromise on the vascular system. Also, GG and AEC is used as an adjunct to mono- and combo-therapies including fibrates, and in particular, in treatment regimens used in patients with prediabetes and diabetes, and hypertriglyceridemia.

### Example 8 (for reference)

### PPAR ACTIVATION

Unexpectedly the triglyceride (TG) dropped (20-30%) in the first 3 months for patients on GG and AEC. Table 3 compares the data of lipid management of normal weight and overweight/obese subjects. The cholesterol management (i.e., TC and LDL) improved in both groups and the TG dropped again in both groups. The HDL in overweight and normal subjects rose by 4% and 10%, respectively. Though modest, the HDL increased with AEC supplementation. It was clearly documented that AEC effectively treated lipidemia of normal weight and overweight/obese subjects, and particularly the TG dropped.

**Table 5. Effect of annatto extract supplementation on blood triglyceride.***

| | Blood TG level (mg/dL) | | |
|---|---|---|---|
| Subject | Control | After | Change (%) |
| 1 | 228 | 203 | -11.0 |
| 2 | 92 | 100 | 8.7 |
| 3 | 164 | 96 | -41.5 |
| 4 | 180 | 176 | -2.2 |
| 5 | 276 | 205 | -25.7 |
| Mean | 188 | 156 | -17.0 |

| | | | |
|---|---|---|---|
| * Subjects took Annatto extract composition (3 softgels/day containing a total of 20mg GG and 75mg T3) for 2 months. | | | |

### Example 10 (for reference)

### STEROL REGULATORY ELEMENT BINDING PROTEIN-1

The studies showed that AEC containing GG reduced IR, and lipids (Tables 4 and 5) where TG consistently dropped. Therefore, AEC containing GG in general, and the GG in particular, deactivated the transcription factor SREBP-1 expression, and thereby inhibited the *de novo* synthesis of fatty acid and TG in various organs, including liver, adipose and skeletal muscle. Adminstration of AEC containing GG simultaneously deactivates SREBP-1 and activates PPAR, which controls FFA/TG regulation in concert in both the metabolism (anabolism and catabolism) and synthesis.

### Example 11 (for reference)

### OTHER USES

GG is used as an adjunctive when a patient is on medication (e.g. statin, bisphosphonate, cyclosporine, fibrate, FTI, niacin, warfarin/coumadin, antifungal, and antibiotic) or any combination of medications thereof.

GG is used to prevent "pill esophagitis" where GG is an excipient in the film-coat of compressed tablets, softgel gelatin, hard gel two-piece gelatin, beads, granules, and liquid coats.

GG is used as a preventative to promote general upper GI health.

GG is administrated to patients who are on combined cortico steroid and bisphosphonate medications.

GG is used to promote general skin health and healing via prenylation of epithelial cells.

## Claims

1. A composition comprising tocotrienols and geranyl geraniols, obtainable from a by-product oily phase of annatto colorant from annatto seeds, wherein the concentration of annatto colorant in the by-product is less than 2% by weight where the tocotrienols are essentially in delta and gamma isomer forms and where the tocotrienols have a delta-to-gamma isomer ratio between 1:100 and 100:1 and where the geranyl geraniols include cis and trans isomer forms and where the geranyl geraniols have a trans-to-cis isomer ratio between 1:100 and 100:1, for use in the decrease in the blood level of the triglyceride which has an effect selected from the group consisting of reversal of insulin resistance, metabolic syndrome, prediabetes, diabetes and diabetes-related cardiovascular disease.

2. The composition for use according to claim 1 for use in a method to reverse insulin resistance.

## Patentansprüche

1. Eine Zusammensetzung, umfassend Tocotrienole und Geranylgeraniole, erhältlich aus einer öligen Phase als Nebenprodukt eines Annatto-Färbemittels aus Annattosamen, wobei die Konzentration an Annatto-Färbemittel in dem Nebenprodukt weniger als 2 Gew.-% beträgt, wobei die Tocotrienole im Wesentlichen in delta- oder gamma-Isomerformen vorliegen und wobei die Tocotrienole ein delta-zu-gamma-Isomerverhältnis zwischen 1:100 und 100:1 aufweisen und wobei die Geranylgeraniole cis- und trans-Isomerformen beinhalten und wobei die Geranylgeraniole ein trans-zucis-Isomerverhältnis zwischen 1:100 und 100:1 aufweisen, zur Verwendung bei der Senkung der Triglycerid-Konzentration im Blut, was eine Wirkung ausgewählt aus der Gruppe bestehend aus Umkehr der Insulinresistenz, des metabolischen Syndroms, von Prädiabetes, von Diabetes und mit Diabetes in Zusammenhang stehender Herz-Kreislauf-Erkrankung hat.

2. Die Zusammensetzung zur Verwendung nach Anspruch 1 zur Verwendung in einem Verfahren zur Umkehr der Insulinresistenz.

## Revendications

1. Composition comprenant des tocotriénols et des géranylgéraniols, pouvant être obtenue à partir d'un sous-produit en phase huileuse du colorant rocou à partir de graines de rocou, dans laquelle la concentration de colorant rocou dans le sous-produit est de moins de 2% en poids, où les tocotriénols sont essentiellement sous formes d'isomères delta et gamma et où les tocotriénols ont un ratio d'isomères delta sur gamma entre 1:100 et 100:1 et où les géranylgéraniols incluent des formes d'isomères cis et trans et où les géranylgéraniols ont un ratio d'isomères trans sur cis entre 1:100 et 100:1, pour une utilisation dans la diminution de niveau sanguin de triglycéride qui a un effet choisi dans le groupe constitué de l'inversion de la résistance à l'insuline, du syndrome métabolique, du prédiabète, du diabète et d'une maladie cardiovasculaire liée au diabète.

2. Composition pour une utilisation selon la revendication 1, pour une utilisation dans une méthode pour inverser la résistance à l'insuline.
